(19) Europäisches Patentamt
European Patent Office
Office européen des brevets

(11) Veröffentlichungsnummer: **0 409 027 A1**

(12) **EUROPÄISCHE PATENTANMELDUNG**

(21) Anmeldenummer: **90113024.5**

(22) Anmeldetag: **07.07.90**

(51) Int. Cl.[5]: **C07D 209/22**, C07D 209/24, A61K 31/40

(30) Priorität: **21.07.89 GB 8916774**

(43) Veröffentlichungstag der Anmeldung: **23.01.91 Patentblatt 91/04**

(84) Benannte Vertragsstaaten: **AT BE CH DE DK ES FR GB GR IT LI LU NL SE**

(71) Anmelder: **BAYER AG**
**D-5090 Leverkusen 1 Bayerwerk(DE)**

(72) Erfinder: **Butler, John E., Dr.**
**Pahlkestrasse 5**
**D-5600 Wuppertal 1(DE)**
Erfinder: **Cuthbert, Nigel James, Dr.**
**Langtree Clarendon Road, Crestwood Great Missenden**
**Bucks HP 16 0PL(GB)**

(54) **Neue Indolderivate, Verfahren zu deren Herstellung und deren Verwendung in Arzneimitteln.**

(57) Die neuen Indolderivate können durch Umsetzung von entsprechenden Indolaldehyden mit Phosphorverbindungen hergestellt werden. Die Indolderivate können als Wirkstoffe in Arzneimitteln, insbesonder als Leukotrienantagonisten verwendet werden.

EP 0 409 027 A1

## ANWENDUNGSGEBIET DER ERFINDUNG:

Die Erfindung bezieht sich auf neue Indolderivate, ein Verfahren zu deren Herstellung und deren Verwendung in Arzneimitteln, insbesondere als Leukotrien-Antagonisten.

## CHARAKTERISTIK DES BEKANNTEN STANDES DER TECHNIK:

Es ist bekannt, dass polymorphonukleare Granulocyten und Mastzellen SRS-A (Slow Reacting Substance of Anaphylaxis) Mediatoren sezernieren, wenn sie z.B. durch Allergene stimuliert werden. SRS-A setzt sich aus den Peptidleukotrienen $LTC_4$, $LTD_4$ und $LTE_4$ zusammen, die aus Arachidonsäure im Zuge des 5-Lipoxygenase-Stoffwechsels gebildet werden. Die Wirkung dieser Leukotriene bei allergischen Erkrankungen und Entzündungserkrankungen kann auf spezifische Rezeptoren auf den Zielzellen (z.B. glatte Muskelzellen) zurückgeführt werden. Es ist darüber hinaus bekannt, dass bei Veränderungen der Struktur der Leukotriene (z.B. partielle Sättigung der Doppelbindungen oder Veränderungen in den, oder Eliminationen der Peptidseitenketten) eine partielle agonistische oder antagonistische Wirkung auftreten kann [vgl. John H. Musser et al., Agents and Actions 18, 332-341 (1986); John G. Gleason et al, J. Med. Chem. 30, (6), 969-961 (1987)].

Es ist darüber hinaus bekannt, dass Derivate der Tetrahydrocarbazolessigsäure und 1-Carboxyalkylindole und -indazole leukotrien-antagonistische Wirkung haben [vgl. US-A-4 783 479 und EP-A-179 019].

## DARLEGUNG DES WESENS DER ERFINDUNG:

Neu Indolderivate der allgemeinen Formel (I)

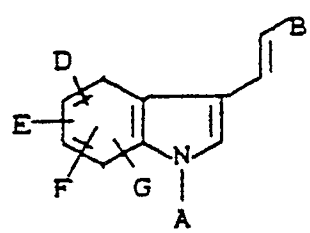

(I)

in der

A - ein geradkettiges oder verzweigtes Alkyl mit bis zu 12 Kohlenstoffatomen bezeichnet, das gegebenenfalls substituiert ist durch Cycloalkyl mit 3 bis 8 Kohlenstoffatomen, Halogen, Hydroxyl, Carboxyl, Cyano, geradkettiges oder verzweigtes Alkoxy oder Alkoxycarbonyl mit jeweils bis zu 10 Kohlenstoffatomen oder durch eine Gruppe der Formel $-CO-NH-SO_2-R^1$, in der

$R^1$ Aryl mit 6 bis 10 Kohlenstoffatomen bezeichnet, das gegebenenfalls einfach bis dreifach substituiert ist durch identische oder verschiedene Substituenten aus der Gruppe, umfassend Carboxyl, Halogen, Cyano, Nitro oder durch geradkettiges oder verzweigtes Alkyl, Alkoxy oder Alkoxycarbonyl mit jeweils bis zu 10 Kohlenstoffatomen,

oder durch Aryl mit 6 bis 10 Kohlenstoffatomen oder durch eine 5- bis 7-gliedrige gesättigte oder ungesättigte heterozyklische Gruppe mit bis zu 4 Heteroatomen, aus der Gruppe, umfassend Stickstoff, Schwefel oder Sauerstoff, die ihrerseits einfach bis vierfach substituiert sind durch identische oder verschiedene Substituenten aus der Gruppe, umfassend Carboxyl, geradkettiges oder verzweigtes Alkyl, Alkylthio, Alkoxy oder Alkoxycarbonyl mit jeweils bis zu 10 Kohlenstoffatomen, oder durch eine Gruppe der Formel $-NH-R^2$, in der

$R^2$ Wasserstoff, geradkettiges oder verzweigtes Alkyl oder Acyl mit jeweils bis zu 8 Kohlenstoffatomen bezeichnet,

B - geradkettiges oder verzweigtes Alkyl, Alkenyl oder Alkinyl mit 12 bis 18 Kohlenstoffatomen bezeichnet, das gegebenenfalls substituiert ist durch Halogen, Mercapto, geradkettiges oder verzweigtes Alkoxy, Acyl oder Alkoxycarbonyl mit jeweils bis zu 10 Kohlenstoffatomen oder durch Aryl mit 6 bis 10 Kohlenstoffatomen, das seinerseits einfach bis vierfach substituiert sein kann durch identische oder verschiedene

Substituenten aus der Gruppe, umfassend Halogen, Nitro, Cyano, Carboxyl, Hydroxyl, oder durch eine Gruppe der Formel $-Y-(CH_2)_n-X-R^3$, in der

X und Y identisch oder verschieden sind, Sauerstoff oder Schwefel, oder eine direkte Bindung bezeichnen,

n eine Zahl 1, 2, 3, 4, 5 oder 6 bezeichnet und

$R^3$ Aryl mit 6 bis 10 Kohlenstoffatomen bezeichnet, das gegebenenfalls substituiert sein kann durch Hydroxyl, Carboxyl, Nitro, Cyano, Halogen oder durch geradkettiges oder verzweigtes Alkyl, Acyl, Alkoxy oder Alkoxycarbonyl mit jeweils bis zu 10 Kohlenstoffatomen,

oder

B - Aryl mit 6 bis 10 Kohlenstoffatomen bezeichnet, das gegebenenfalls einfach bis dreifach substituiert sein kann durch identische oder verschiedene Substituenten aus der Gruppe umfassend Halogen, Nitro, Hydroxyl oder durch eine Gruppe der Formel $-Y-(CH_2)n-X-R^3$, in der

X, Y, $R^3$ und n die vorstehenden Bedeutungen haben,

D, E, F und G sind identisch oder verschieden und bezeichnen:

- Wasserstoff, Halogen, Cyano, Hydroxyl oder Carboxyl,
- geradkettiges oder verzweigtes Alkyl, Alkoxy, Alkoxycarbonyl oder Acyl mit jeweils bis zu 10 Kohlenstoffatomen oder Cycloalkyl mit 3 bis 8 Kohlenstoffatomen,
- eine 5- bis 7-gliedrige, gesättigte oder ungesättigte heterozyklische Gruppe mit bis zu 4 Heteroatomen aus der Gruppe, umfassend Stickstoff, Schwefel oder Sauerstoff, oder
- eine Gruppe der Formel $-CO-NH-SO_2-R^1$, in der

$R^1$ die vorerwähnte Bedeutung hat,

und deren physiologisch annehmbaren Salze.

Im Zusammenhang mit der vorliegenden Erfindung sind physiologisch annehmbare Salze bevorzugt. Physiologisch annehmbare Salze der substituierten Indolderivate können Salze der erfindungsgemässen Substanzen mit Carbonsäuren oder Sulfonsäuren sein. Besonders bevorzugte Salze sind z.B. diejenigen mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalidisulfonsäure, Essigsäure, Propionsäure, Milchsäure, Weinsteinsäure, Zitronensäure, Fumarsäure, Maleinsäure oder Benzoesäure.

Erfindungsgemässe Salze sind Salze der monovalenten Metalle, wie z.B. Alkalimetalle, und die Ammoniumsalze. Natriumsalze, Kaliumsalze und Ammoniumsalze sind bevorzugt.

Die erfindungsgemässen Verbindungen können in ihren stereoisomeren Formen vorliegen, die sich entweder wie Bild und Spiegelbild (Enantiomere) oder die sich nicht wie Bild und Spiegelbild (Diastereomere) verhalten. Die Erfindung bezieht sich sowohl auf die Antipoden und die Racemate als auch auf die Mischungen der Diastereomeren. Die Racemate können, ebenso wie die Diastereomeren, in bekannter Weise, z.B. durch Kristallisation, Chromatografie oder Craig-Verteilung in ihre stereoisomereinheitlichen Bestandteile getrennt werden [vgl. E.L. Eliel, Stereochemistry of Carbon Compounds, McGraw Hill, 1962].

Bevorzugte Verbindungen sind solche der allgemeinen Formel (I), in der

A - geradkettiges oder verzweigtes Alkyl mit bis zu 10 Kohlenstoffatomen bezeichnet, das gegebenenfalls substituiert ist durch Cyclopropyl, Cyclopentyl, Cyclohexyl, Fluor, Chlor, Brom, Hydroxyl, Carboxyl, Cyano, geradkettiges oder verzweigtes Alkoxy oder Alkoxycarbonyl mit jeweils bis zu 8 Kohlenstoffatomen, oder durch eine Gruppe der Formel $-CO-NH-SO_2-R^1$, in der

$R^1$ Phenyl oder Naphthyl bezeichnet, wobei jede dieser Gruppen gegebenenfalls einfach oder zweifach substituiert ist durch identische oder verschiedene Substituenten aus der Gruppe, umfassend Carboxyl, Fluor, Chlor, Brom, Cyano, Nitro oder durch geradkettiges oder verzweigtes Alkyl, Alkoxy oder Alkoxycarbonyl mit jeweils bis zu 8 Kohlenstoffatomen,

oder durch Phenyl, Naphthyl, Thienyl, Furyl, Pyridyl, Pyrimidyl, Chinolyl, Isochinolyl, Pyrryl, Imidazolyl, 1,3-Thiazolyl, Tetrazolyl, Oxazolyl, Isoxazolyl, Isothiazolyl, Triazolyl oder Thiadiazolyl, die ihrerseits einfach bis dreifach substituiert sind durch identische oder verschiedene Substituenten aus der Gruppe, umfassend Carboxyl, geradkettiges oder verzweigtes Alkyl, Alkylthio, Alkoxy oder Alkoxycarbonyl mit jeweils bis zu 8 Kohlenstoffatomen, oder durch eine Gruppe der Formel $-NH-R^2$ in der

$R^2$ Wasserstoff oder geradkettiges oder verzweigtes Alkyl oder Acyl mit jeweils bis zu 6 Kohlenstoffatomen bezeichnet,

B - geradkettiges oder verzweigtes Alkyl oder Alkenyl mit jeweils 12 bis 16 Kohlenstoffatomen bezeichnet, wobei jedes gegebenenfalls substituiert ist durch Fluor, Chlor, Brom, Carboxyl, Hydroxyl, geradkettiges oder verzweigtes Alkoxy, Acyl oder Alkoxycarbonyl mit jeweils bis zu 8 Kohlenstoffatomen oder durch Phenyl, das seinerseits einfach bis dreifach substituiert sein kann durch identische oder verschiedene Substituenten aus der Gruppe, umfassend Fluor, Chlor, Brom, Carboxyl, Cyano, Hydroxyl, oder durch eine Gruppe der Formel $-Y-(CH_2)_n-X-R^3$, in der

X und Y identisch oder verschieden sind, Sauerstoff oder Schwefel, oder eine direkte Bindung bezeichnen,
n eine Zahl 1, 2, 3, 4, 5 oder 6 bezeichnet und
$R^3$ Phenyl oder Naphthyl bezeichnet, wobei jedes gegebenenfalls substituiert ist durch Hydroxyl, Carboxyl, Cyano, Fluor, Chlor, Brom, oder durch ein geradkettiges oder ein verzweigtes Alkoxy, Acyl oder Alkoxycarbonyl mit jeweils bis zu 8 Kohlenstoffatomen,
oder
B - Phenyl bezeichnet, das gegebenenfalls einfach oder doppelt substituiert ist durch identische oder verschiedene Substituenten aus der Gruppe umfassend Fluor, Chlor, Brom, Nitro, Hydroxyl oder durch eine Gruppe der Formel $-Y-(CH_2)n-X-R^3$, in der
X, Y, n und $R^3$ die vorstehenden Bedeutungen haben,
D, E, F und G identisch oder verschieden sind und bezeichnen:
- Wasserstoff, Fluor, Chlor, Brom, Cyano, Carboxyl, geradkettiges oder verzweigtes Alkyl, Acyl oder Alkoxycarbonyl mit jeweils bis zu 8 Kohlenstoffatomen, Thienyl, Furyl, Pyridyl, Pyrimidyl, Chinolyl, Isochinolyl, Pyrryl, Imidazolyl, 1,3-Thiazolyl, Tetrazolyl, Oxazolyl, Isoxazolyl, Isothiazolyl, Triazolyl, Thiadiazolyl oder
- eine Gruppe der Formel $-CO-NH-SO_2-R^1$, in der
$R^1$ die vorerwähnte Bedeutung hat,
und deren physiologisch annehmbaren Salze.

Besonders bevorzugte Verbindungen sind diejenigen der allgemeinen Formel (I), in der
A - geradkettiges oder verzweigtes Alkyl mit bis zu 8 Kohlenstoffatomen bezeichnet, das gegebenenfalls substituiert ist durch Carboxyl, Cyano, geradkettiges oder verzweigtes Alkoxycarbonyl mit bis zu 6 Kohlenstoffatomen oder durch eine Gruppe der Formel $-CO-NH-SO_2-R^1$, in der
$R^1$ Phenyl bezeichnet, das gegebenenfalls substituiert ist durch Cyano, Carboxyl oder durch geradkettiges oder verzweigtes Alkyl oder Alkoxycarbonyl mit jeweils bis zu 6 Kohlenstoffatomen,
oder durch Phenyl, Thienyl, Furyl, Pyrryl, 1,3-Thiadiazolyl, Tetrazolyl, Oxazolyl, Isoxazolyl, Isothiazolyl, Triazolyl oder Thiadiazolyl,
B - geradkettiges oder verzweigtes Alkyl oder Alkenyl mit 12 Kohlenstoffatomen bezeichnet, wobei jedes gegebenenfalls substituiert ist durch Carboxyl, Acetyl, Propanoyl, geradkettiges oder verzweigtes Alkoxy oder Alkoxycarbonyl mit jeweils bis zu 6 Kohlenstoffatomen, oder durch Phenyl, das seinerseits einfach oder zweifach substituiert sein kann durch identische oder verschiedene Substituenten aus der Gruppe, umfassend Cyano, Carboxyl oder durch eine Gruppe der Formel $-Y-(CH_2)_nX-R^3$, in der
X und Y identisch oder verschieden sind, Sauerstoff oder Schwefel oder eine direkte Bindung bezeichnen,
n eine Zahl 2, 3 oder 4 bezeichnet
und
$R^3$ Phenyl bezeichnet, das gegebenenfalls substituiert ist durch Hydroxyl, Cyano, Acetyl, Propanoyl, Carboxyl oder durch Alkoxycarbonyl mit bis zu 6 Kohlenstoffatomen, oder
B Phenyl bezeichnet, das substituiert ist durch eine Gruppe der Formel $-Y-(CH_2)_n-X-R^3$, in der
X, Y, n und $R^3$ die vorstehenden Bedeutungen haben,
D, E und F Wasserstoff bezeichnen, und
G - Cyano, Carboxyl, Alkoxycarbonyl mit bis zu 6 Kohlenstoffatomen, Thienyl, Furyl, Tetrazolyl, Triazolyl bezeichnet oder
- eine Gruppe der Formel $-CO-NH-SO_2-R^1$ bezeichnet, in der
$R^1$ die vorstehende Bedeutung hat,
und deren physiologisch annehmbaren Salze.

Die erfindungsgemässen Verbindungen der allgemeinen Formel (I) werden hergestellt durch Umsetzen von Aldehyden der allgemeinen Formel (II)

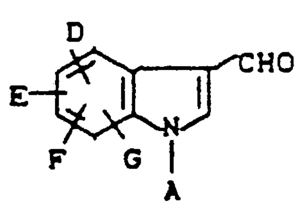

(II)

in der D, E, F, G und A die vorerwähnten Bedeutungen haben, mit Phosphorverbindungen der allgemeinen Formel (III), (IV) und (V)

$\overset{+}{P}(R^4)_3CH_2B[Z]^-$ (III)

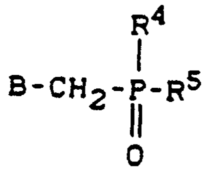

(IV)

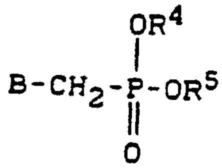

(V)

in denen
B - die vorstehende Bedeutung hat,
$R^4$ und $R^5$ identisch oder verschieden sind und $(C_{1-6})$-Alkyl oder Phenyl bezeichnen, und
Z - Chlor, Brom, Jod oder das Tosylatanion bezeichnet,
in inerten Lösungsmitteln, geeignetenfalls in Anwesenheit von Basen und, im Falle der Säuren, durch Hydrolyse der Ester durch herkömmliche Methoden.

Das Verfahren wird beispielsweise durch die folgenden Formeln veranschaulicht:

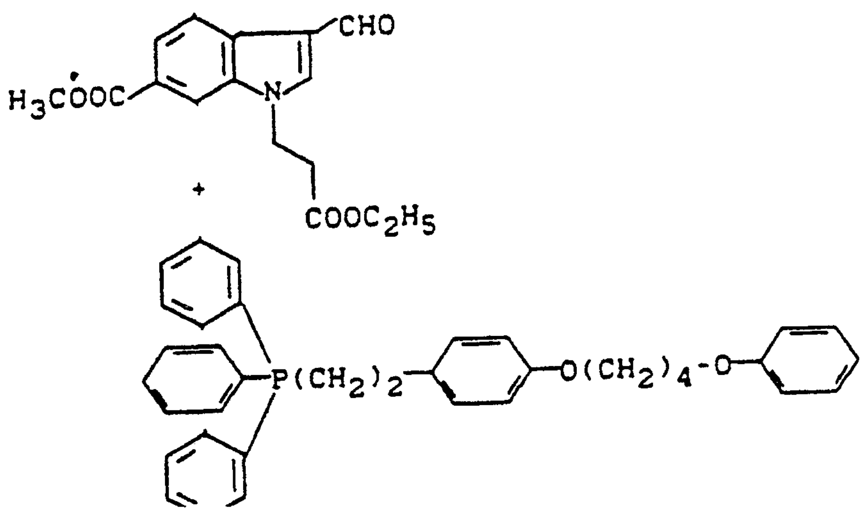

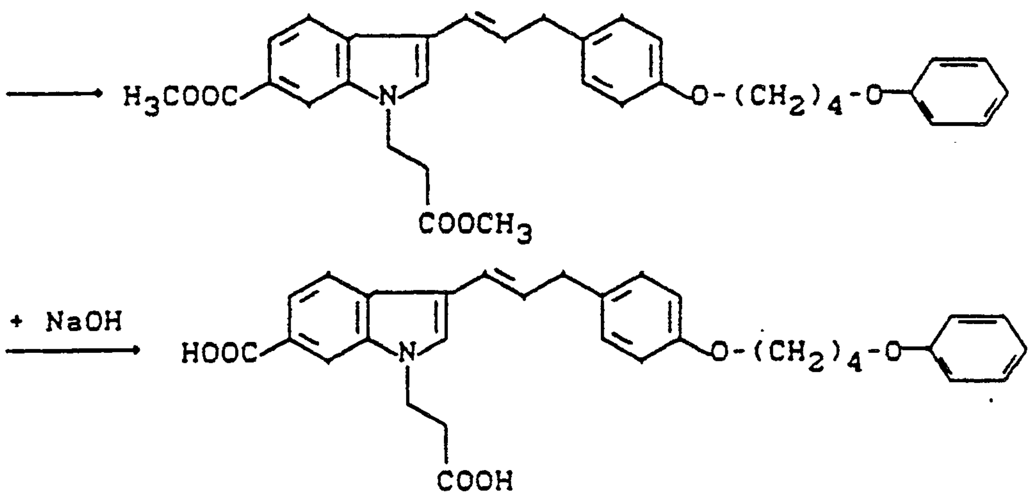


Geeignete Lösungsmittel sind die herkömmlichen organischen Lösungsmittel, die sich unter den Reaktionsbedingungen nicht verändern. Ether, wie z.B. Diethylether, Butylmethylether, Dioxan, Tetrahydrofuran, Glykoldimethylether oder Ethylenglykoldimethylether, oder Kohlenwasserstoffe, wie z.B. Benzol, Toluol oder Xylol, oder Amide wie z.B. Dimethylformamid oder Hexamethylphosphoramid, oder 1,3-Dimethylimidazolidin-2-on, 1,3-Dimethyl-tetrahydro-pyridin-2-on oder Dimethylsulfoxid sind bevorzugt. Es ist ebenso möglich, Mischungen der vorerwähnten Lösungsmittel zu verwenden. Tetrahydrofuran und Dimethylformamid sind bevorzugt.

Geeignete Basen für die Wittig-Reaktion sind herkömmliche organische und anorganische Basen. Vorzugsweise umfassen diese Alkalimetallhydride, wie z.B. Natriumhydrid oder Kaliumhydrid, oder Alkalimetallalkoxide, wie z.B. Natriummethoxid, Natriumethoxid, Kaliummethoxid, Kaliumethoxid oder Kalium-tert.-butoxid, oder Amide, wie z.B. Natrium amid oder Lithiumdiisopropylamid, oder organische Lithiumverbindungen, wie Phenyllithium, Butyllithium oder Methyllithium, oder Natriumhexamethylsilazan. Kalium-tert.-butoxid, Phenyllithium oder Butyllithium sind bevorzugt.

Die Phosphorverbindungen (III), (IV) und (V) werden im allgemeinen in einer Menge von 1 bis 2 Molen verwendet, vorzugsweise in molaren Mengen, bezogen auf 1 Mol des Aldehyds (II). Die Base wird im allgemeinen in einer Menge von 1 bis 5 Molen verwendet, vorzugsweise von 1 bis 2 Molen, in bezug auf 1 Mol der Phosphorverbindungen.

Die Wittig-Reaktion wird im allgemeinen in einem Temperaturbereich von -80 bis +40° C durchgeführt, vorzugsweise von -80 bis +10° C bei atmosphärischem, erhöhten oder verminderten Druck, vorzugsweise bei atmosphärischem Druck.

Die Wittig-Reaktion kann entweder in einer zweistufigen Reaktion oder in einem einstufigen Verfahren durchgeführt werden: In der zweistufigen Reaktion werden zuerst die Phosphorane $[(R^4)_3P=CH_2\text{-}B]$ aus den entsprechenden Phosphoniumsalzen unter der Wirkung von Basen hergestellt und diese werden sodann mit den Aldehyden (II) umgesetzt. In der einstufigen Reaktion, die bevorzugt ist, werden die Salze der Verbindungen gemäss Formel (III) direkt mit den Aldehyden (II) in Anwesenheit einer Base umgesetzt.

Geeignete Basen für die Hydrolyse sind die herkömmlichen anorganischen Basen. Diese schliessen vorzugsweise Alkalimetallhydroxide oder Erdalkalimetallhydroxide ein, wie z.B. Natriumhydroxid, Kaliumhydroxid, Lithiumhydroxid oder Bariumhydroxid, oder Alkalimetallkarbonate, wie Natrium karbonat oder Kaliumkarbonat. Vorzugsweise werden Natriumhydroxid oder Lithiumhydroxid eingesetzt.

Geeignete Lösungsmittel für die Hydrolyse sind Wasser oder die für Hydrolyse herkömmlicherweise verwendeten organischen Lösungsmittel. Diese schliessen bevorzugt Alkohole mit ein, wie Methanol, Ethanol, Propanol, Isopropanol oder Butanol, oder Ether, wie Tetrahydrofuran oder Dioxan, oder Dimethylformamid oder Dimethylsulfoxid. In besonders bevorzugter Weise werden Alkohole, wie Methanol, Ethanol, Propanol oder Isopropanol, verwendet. Es ist ebenso moglich, Mischungen der vorerwähnten Lösungsmittel zu verwenden.

Die Hydrolyse wird im allgemeinen in einem Temperaturbereich von 0 bis 100° C ausgeführt, vorzugsweise bei Raumtemperatur.

Im allgemeinen wird die Hydrolyse bei atmosphärischem Druck ausgeführt. Es ist aber ebenso möglich, bei vermindertem Druck oder bei erhöhtem Druck zu arbeiten (z.B. von 0,5 bis 5 bar).

Wenn die Hydrolyse ausgeführt wird, wird die Base im allgemeinen in einer Menge von 1 bis 3 Molen

angewandt, vorzugsweise von 1 bis 1,5 Molen, in bezug auf 1 Mol des Esters oder des Lactons. In besonders bevorzugter Weise werden molare Anteile der Reaktanten verwendet.

Wenn die Reaktion ausgeführt wird, werden in dem ersten Schritt die Salze der erfindungsgemässen Verbindungen als Zwischenprodukte hergestellt, die isoliert werden können.

Die erfindungsgemässen Säuren werden durch Behandeln der Salze mit herkömmlichen anorganischen Säuren erhalten. Diese schliessen vorzugsweise Mineralsäuren mit ein, wie z.B. Chlorwasserstoffsäure, Bromwasserstoffsäure, Schwefelsäure oder Phosphorsäure. Es hat sich als vorteilhaft erwiesen, in der Herstellung der Carbonsäuren die basische Reaktionsmischung aus der Hydrolyse in einem zweiten Schritt ohne Isolierung der Salze anzusäuern. Die Säuren können sodann in herkömmlicher Weise isoliert werden.

Die Aldehyde der allgemeinen Formel (II) sind neu und können hergestellt werden durch

Umsetzen der Verbindungen der allgemeinen Formel (VI)

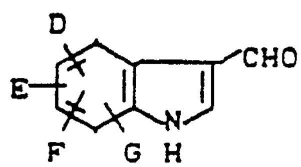

(VI)

in der D, E, F und G die vorstehenden Bedeutungen haben, mit Verbindungen der allgemeinen Formel (VII)

Hal-A (VII)

in der Hal Fluor, Chlor, Brom oder Jod bezeichnet und A die vorerwähnte Bedeutung hat, in inerten Lösungsmitteln, geeignetenfalls in Anwesenheit einer Base und, im Falle der Säuren, durch Hydrolyse der Ester durch die vorerwähnten Methoden.

Bei der Ausführung der Reaktion können die in Zusammenhang mit der Wittig-Reaktion vorerwähnten Lösungsmittel und Basen verwendet werden.

Die Verbindungen der allgemeinen Formel (VI) sind als solche bekannt und können nach bekannten Methoden hergestellt werden, z.B. durch Umsetzen der entsprechenden Indole mit Phosphoroxychlorid in Dimethylformamid [vgl. H. Büttcher, R. Gericke, Liebigs Ann. Chem. 1988, 749-752; Organikum (Organic Compounds), S. 405, VEB Deutscher Verlag der Wissenschaften, Berlin 1977].

Die Verbindungen der allgemeinen Formeln (III), (IV) und (V) sind ebenso bekannt und können durch herkömmliche Methoden hergestellt werden [J.C. Buck, F, Ellis, P. North, Tetrahedron Letters 1982, 4161-4162].

Die Verbindungen der allgemeinen Formel (VII) sind als solche bekannt und können nach herkömmlichen Methoden hergestellt werden. Als Beispiele können angeführt werden: Ethyl-3-brompropionat und Ethylchlormethylbenzoat [vgl. Beilstein, Bd. II, S. 256, Beilstein Bd. IX, S. 458].

Die erfindungsgemässen Verbindungen zeigen ein unvorhersehbares nützliches Spektrum an pharmakologischer Wirkung. Sie blockieren die spezifischen Rezeptoren der Leukotriene auf den Zielzellen (z.B. Muskelzellen) und wirken daher als Leukotrien-Antagonisten.

Sie können deshalb in Medikamenten zur Behandlung und Prophylaxe allergischer Erkrankungen und Entzündungserkrankungen verwendet werden, wie z.B. Allergien, Asthma, Bronchitis, Emphysem, Schocklunge, Lungenhochdruck, Entzündun gen, Ödeme, Thrombosen und Thromboembolien, Ischämie (periphere, kardiale oder zerebrale Zirkulationsstörungen), Herz- oder Niereninfarkte, Herzrythmusstörungen, Angina pectoris, Arteriosklerose, in Gewebstransplantaten, Dermatosen, wie Psoriasis, Metastasen und für die Cytoprotektion im Gastrointestinaltrakt.

AUSFÜHRUNGSBEISPIELE:

BESCHREIBUNG DES TESTS:

(1) Präparation:

Meerschweinchen werden durch einen Schlag auf den Kopf getötet und die Luftröhre wird in eine Tyrode-Lösung gegeben (Zusammensetzung in mM: NaCl 137, $MgCl_2$ 2,1, KCl 2,7, $NaH_2PO_4$ 0,5, $CaCl_2$ 2,4, $NaHCO_3$ 11,9, D-Glukose 9,2), die Indomethacin ($3 \times 10^{-6}$ M) enthält. Die Luftröhre wird longitudinal

geöffnet und alternierend transversal über 3/4 der gesamten Organbreite geschnitten. Die Präparation wird als eine Zick-Zack-Kette aufgefaltet und in ein 10 ml-Organbad gegeben, das eine Tyrode-Lösung und einen Indomethacin-Zusatz (3 x $10^{-6}$ M) enthält, und die Lösung wird bei einer Temperatur von 37° C mit Sauerstoff mit 5 % $CO_2$ belüftet. Die Kontraktion wurde mit Hilfe eines isotonischen Hugo Sachs-Transducers unter Verwendung einer Beladung von 250 bis 300 mg überwacht.

(2) Experiment:

Nach der Equilibrierung in dem Organbad wird die maximale Kontraktion mit $10^{-4}$ und 3 x $10^{-4}$ M Histamin bestimmt. Das Histamin wird ausgewaschen und die Tyrode-Lösung wird durch eine Tyrode-Lösung ersetzt, die Indomethacin, L-Serinborat (45 mM) und L-Cystein (10 mM) enthält. Nach der neuerlichen Equilibrierung werden 10 µl Ethanol zu einer der vier Präparationen als Kontrolle zugegeben. Die erfindungsgemässen Verbindungen werden in Konzentrationen von $10^{-11}$ bis $10^{-5}$ M zu den anderen drei Präparationen in jeweils zunehmenden Mengen zugegeben. 15 Minuten nach der letzten Zugabe der erfindungsgemässen Verbindungen oder des Ethanols erhält man eine ansteigende Konzentrations-/Wirkungskurve für $LTD_4$ ($10^{-10}$ bis $10^{-6}$ M). Nach Erreichen der maximalen $LTD_4$-Konzentration wird das Gewebepräparat verworfen.

(3) Ergebnisse:

Die Konzentrationen werden an dem histamininduzierten Maximum für jede Präparation standardisiert. Die Reaktion auf die erfindungsgemässen Verbindungen $LTD_4$ und $LTD_4$ zusammen mit den erfindungsgemässen Verbindungen wird sodann in Prozent als die Wirkung auf das $LTD_4$-Maximum in der entsprechenden Kontrollpräparation angegeben. Die $EC_{50}$-Werte (50 % der maximalen $LTD_4$-Konzentration) der Experimente und der Kontrollexperimente werden durch die durchschnittliche Quadratwurzelabweichungsmethode bestimmt. Die Ergebnisse werden verwendet, um einen $pk_B$-Wert zu berechnen, um somit das quantitative Ausmass der entsprechenden antagonistischen Wirkung festzustellen.

| Beispiel Nr. | $pk_B$ |
|---|---|
| 5 | 8,1 |
| 7 | 6,1 |
| 17 | 7,3 |

Die neuen, aktiven Verbindungen können in bekannter Weise durch Verwendung von nicht-toxischen, pharmazeutisch geeigneten Trägern oder Lösungsmitteln in die herkömmlichen Formulierungen überführt werden, wie Tabletten, Kapseln, beschichtete Tabletten, Pillen, Granulate, Aerosole, Sirup, Emulsionen, Suspensionen und Lösungen. In diesem Zusammenhang soll die therapeutisch wirksame Verbindung in jedem Fall in einer Konzentration von ungefähr 0,5 bis 90 Gew.%, vorzugsweise von 10 bis 70 Gew.%, in der gesamten Mischung vorhanden sein, d.h. in Mengen, die ausreichend sind, den angegebenen Dosisbereich zu erreichen.

Die Formulierungen werden z.B. durch Strecken der aktiven Verbindung unter Verwendung von Lösungsmitteln und/oder Trägern, gegebenenfalls unter Verwendung von Emulgatoren und/oder Dispersionsmitteln hergestellt, wenn, z.B. im Fall der Verwendung von Wasser als Verdünnungsmittel, geeignete organische Lösungsmittel als Hilfslösungsmittel verwendet werden können.

Beispiele für diese Hilfsstoffe sind Wasser, nicht-toxische, organische Lösungsmittel, wie Paraffine (z.B. Mineralölfraktionen), Pflanzenöle (z.B. Erdnuss-/Sesamöl), Alkohole (z.B. Ethylalkohol, Glycerin), Glykole (z.B. Pro pylenglykol, Polyethylenglykol), feste Träger, wie natürliche Bodenmineralien (z.B. Kaoline, Aluminiumoxide, Talk, Kreide), synthetische Bodenmineralien (z.B. hochdisperse Siliziumoxide, Silicate), Zucker (z.B. Sacharose, Lactose und Dextrose), Emulgatoren (z.B. Polyoxyethylen-Fettsäureester, Polyoxyethylen-Fettalkoholether, Alkylsulfonate und Arylsulfonate), Dispergiermittel (z.B. Ligninsulfite aus Abwasser, Methylzellulose, Stärke und Polyvinylpyrrolidon) und Gleitmittel (z.B. Magnesiumstearat, Talk, Stearinsäure und Natriumlaurylsulfat).

Die Darreichung kann in einer üblichen Form ausgeführt werden, vorzugsweise oral oder parenteral, insbesondere perlingual oder intravenös. Im Fall der oralen Darreichung können die Tabletten natürlich auch Zusätze enthalten, wie Natriumzitrat, Kalziumkarbonat und Dikalziumphosphat, zusammen mit verschiedenen Zusätzen, wie Stärke, vorzugsweise Kartoffelstärke, Gelatine und dergleichen, zusätzlich zu den vorerwähnten Trägern. Darüber hinaus können Gleitmittel, wie Magnesiumstearat, Natriumlaurylsulfat und Talk, zusätzlich für die Tablettierung verwendet werden. Im Falle der wässrigen Suspensionen und/oder Elexiere, wie sie für die orale Darreichung bestimmt sind, können eine Vielzahl von Geschmacksverstärkern oder Färbemitteln den aktiven Verbindungen zusätzlich zu den vorerwähnten Hilfsstoffen zugegeben werden.

Im Fall der parenteralen Darreichung können Lösungen der aktiven Verbindung unter Verwendung geeigneter flüssiger Trägermaterialien verwendet werden.

Im allgemeinen hat es sich bei der intravenösen Darreichung als vorteilhaft erwiesen, Mengen von ungefähr 0,01 bis 1o mg/kg darzureichen, vorzugsweise ungefähr 0,01 bis 5 mg/kg Körpergewicht, um effektive Wirkungen zu erreichen. Bei der oralen Darreichung ist die Dosierung im allgemeinen ungefähr 0,1 bis 200 mg/kg, vorzugsweise 1 bis 100 mg/kg Körpergewicht.

Im Gegensatz dazu kann es notwendig sein, von den vorerwähnten Mengen abzuweichen, abhängig vom Körpergewicht oder von der gewählten Darreichungsform, vom individuellen Verhalten in bezug auf das Medikament, von der Art ihrer Formulierung und vom Zeitpunkt oder Zeitintervall der Darreichung. Es kann somit in gewissen Fällen ausreichend sein, mit weniger als der vorerwähnten minimalen Menge auszukommen, während in anderen Fällen das vorerwähnte obere Limit überstiegen werden muss. Im Falle der Darreichung von grösseren Mengen kann es angezeigt sein, diese auf mehrere individuelle Dosen, verteilt über den Tag, aufzuteilen.

Die erfindungsgemässen Indole können sowohl in der Humanmedizin als auch in der Veterinärmedizin verwendet werden.

BEISPIELE FÜR DIE HERSTELLUNG

BEISPIEL 1

Methyl-6-indolcarboxylat

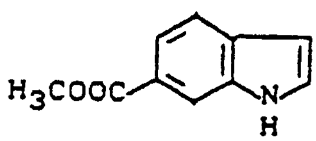

16,7 g (0,060 mol) Methyl-4-(2-dimethylaminoethenyl)-3-nitrobenzoat (hergestellt nach der allgemeinen Arbeitsvorschrift von L.F. Tietze und Th. Eicher, "Reaktionen und Synthesen" S. 172, Thieme, Stuttgart 1981) wurden in 300 ml Tetrahydrofuran, das 1,1 g Palladiumschwarz (10 %) enthält, hydriert. Die Lösung wurde für 2 Stunden über Kieselgur filtriert und der Katalysator wurde mit 50 ml Tetrahydrofuran gewaschen. Das Lösungsmittel wurde im Vakuum entfernt. Um eine geringe Menge von Verunreinigungen (Methyl-3-amino-4-methylbenzoat) zu entfernen, wurde das Produkt nacheinander mit 10 %-iger Chlorwasserstoffsäure, Wasser und konzentrierter Natriumchloridlösung gewaschen, über Magnesiumsulfat getrocknet und in einem Rotationsverdampfer konzentriert.

Ausbeute: 9,9 g gelber Kristalle (94 % der Theorie)

Rf ($CH_2Cl_2$) = 0,38

Rf ($CH_2Cl_2$, 5 % MeOH) = 0,53

BEISPIEL 2

Methyl-3-formyl-6-indolcarboxylat

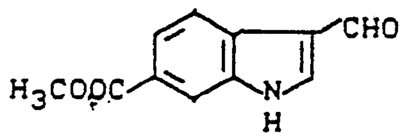

4,3 ml Phosphoroxychlorid wurden tropfenweise unter Rühren und Kühlen (0 °C) zu 14 ml Dimethylformamid zugegeben und die Mischung wurde für weitere 15 Minuten bei 0 °C gerührt. Danach wurde eine Lösung von 8,6 g (0,049 mol) der in Beispiel 1 erhaltenen Verbindung in 13 ml Dimethylformamid tropfenweise bei Raumtemperatur zugegeben, wobei die Temperatur nicht über 30 °C stieg. Die Lösung wurde für weitere 3 Stunden bei 35 °C gerührt, tropfenweise zu Eiswasser zugegeben und auf einen pH-Wert von 7 unter Verwendung einer 2 N Natriumhydroxidlösung eingestellt. Die Lösung wurde mit Methylenchlorid gewaschen und erneut auf einen pH-Wert von 1 unter Verwendung einer 2 N Salzsäure eingestellt. Über Nacht bildete sich ein Niederschlag aus, der abgesaugt und getrocknet wurde.
Ausbeute: 6,4 g eines weissen Pulvers (64 % der Theorie).
Rf (9:1, $CH_2Cl_2$/MeOH) = 0,54
Rf (95:5, $CH_2Cl_2$/MeOH) = 0,38

BEISPIEL 3

Methyl-1-(2-ethoxycarbonyl)ethyl-3-formyl-6-indolcarboxylat

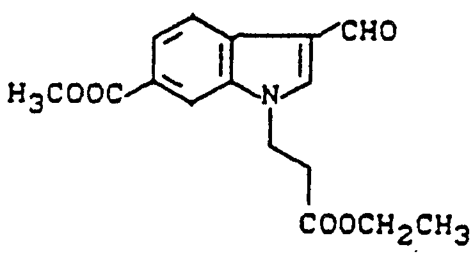

3,9 g (0,019 mol) Indolaldehyd, die in Beispiel 2 erhaltene Verbindung, 4,0 g (0,022 mol) Ethyl-3-brompropionat, 5,3 g (0,038 mol) Kaliumkarbonat und 30 ml Dimethylform amid wurden bei 100 °C für 3 Stunden gerührt. Das Lösungsmittel wurde im Vakuum entfernt und der Rückstand wurde mit 50 ml Wasser gerührt und auf 5 °C abgekühlt. Das weisse, amorphe Präzipitat wurde durch Absaugen abfiltriert, mit Wasser gewaschen und im Vakuum über Phosphorpentoxid getrocknet.
Ausbeute: 5,3 g (90 % der Theorie)
Schmelzpunkt: 129 °C
Rf (9:1, $CH_2Cl_2$/MeOH) = 0,85

BEISPIEL 4

Methyl-3-[2-[4-(4-phenoxybutoxy)phenyl]-2-(Z)-ethenyl]-6-indolcarboxylat (4a) und das E-Isomere (4b)

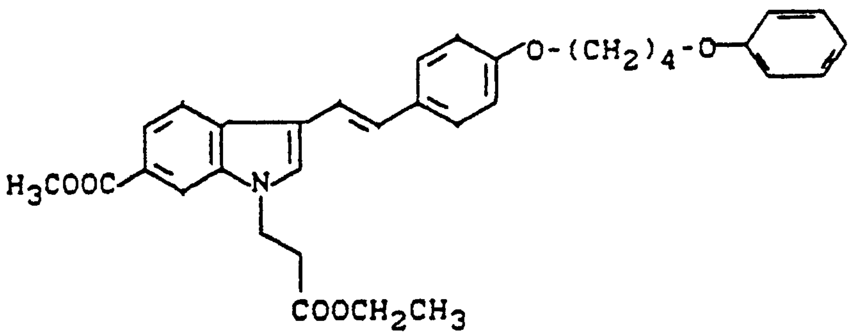

70 ml (0,026 mol) frisch hergestelltes Lithiumdiisopropylamid wurden tropfenweise unter Stickstoff bei -78° C in Tetrahydrofuran (0,37 mol) zu einer Lösung von 15,3 g (0,026 mol) 4-(4-Phenoxybutoxy)-benzyltriphenylphosphoniumbromid in 25 ml absolutem Tetrahydrofuran zugegeben. Die Reaktionslösung wurde bei -20° C für 30 Minuten gerührt und auf -78° C abgekühlt. Eine Lösung von 7,1 g (0,023 mol) Indolaldehyd der Verbindung von Beispiel 3 in 100 ml absolutem Tetrahydrofuran wurde sodann tropfenweise über einen Zeitraum von 30 Minuten zugegeben. Die Mischung wurde bei Raumtemperatur für 2,5 Stunden gerührt und mit Wasser verdünnt, und die Lösung wurde mehrere Male unter Verwendung von Ether extrahiert. Die organische Phase wurde mit einer gesättigten Natriumhydrogenkarbonatlösung gewaschen, unter Verwendung von Magnesiumsulfat getrocknet und abgedampft. Der Rückstand wurde über 300 g Silicagel unter Verwendung von Petrolether/Ether 1:1 chromatografiert. In dieser Weise wurde eine Mischung der cis- und trans-Isomeren erhalten, die nochmals über 800 g Silicagel unter Verendung von 1:1 Petrolether/Ether chromatografiert wurde. In dieser Weise wurden drei Fraktionen erhalten, von denen die erste reines cis- und die dritte reines trans-Isomer enthielt. Nach der Chromatografie der gemischten Fraktionen (2,0 g) über 400 g Silicagel wurden zwei reine Isomere erhalten.

Ausbeute: 5,43 g des cis-Isomers (4a) (43 % der Theorie)

3,99 g des trans-Isomers (4b) (32 % der Theorie)

Rf (Petrolether/Ether 1:1) cis-Isomer = 0,30

trans-Isomer = 0,20

$^1$H-NMR (DMSO) delta = 6,55 (-CH=CH-cis, AB-System, I = 12,5 Hz), 7,20 (-CH=CH-trans, AB-System, I = 16,0 Hz).

BEISPIEL 5

1-(2-Ethoxycarbonyl)-ethyl-3-( [2-[4-(4-phenoxybutoxy)phenyl]-2-(Z)-ethenyl]-6-indolcarbonsäure

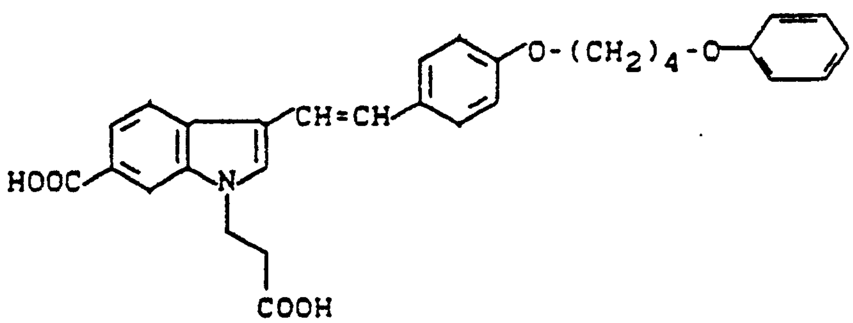

7,9 g (0,015 mol) Methylindolcarboxylat, die Verbindung von Beispiel 4a, wurde in 90 ml Tetrahydrofuran gelöst, 15 ml Methanol wurden zugegeben und die Mischung wurde auf 0° C abgekühlt. 20 ml einer 2 N Natriumhydroxidlösung wurden tropfenweise zugegeben und die Mischung erreichte über Nacht Raumtemperatur. Sie wurde mit Wasser verdünnt und unter Verwendung von 2 N Schwefelsäure auf einen pH-Wert von 6 angesäuert. Die trübe Lösung wurde mehrere Male mit Ethylacetat extrahiert, sodann mit Wasser gewaschen und über Magnesiumsulfat getrocknet. Es wurden 6,80 g (94 % der Theorie) blassgelber Kristalle aus der eingedampften Lösung in Ethylacetat erhalten.

Schmelzpunkt: 185 °C
Rt (RP-HPLC): 7,990
UV ($CH_3CN$): lambda max: 208, 248, 293 und 323 nm.

Die in der folgenden Tabelle dargestellten Beispiele 6 bis 16 wurden in einer zu Beispiel 5 analogen Weise hergestellt.

TABELLE 1

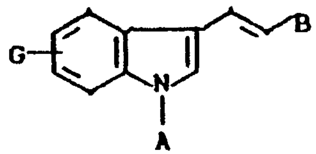

| Beispiel Nr. | A | B | G | Doppelbindungsisomer | Rf oder Schmelzpunkt (°C) |
| --- | --- | --- | --- | --- | --- |
| 6 | $-(CH_2)_2-COOH$ | 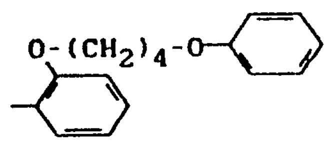 | 6-COOH | trans | 0,42 (A) |
| 7 | $-(CH_2)_2-COOH$ | 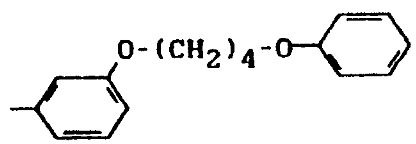 | 6-COOH | cis | 0,45 (A) |
| 8 | $-(CH_2)_2-COOH$ | 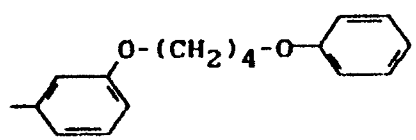 | 6-COOH | trans | 0,30 (A) |
| 9 | $-(CH_2)_2-COOH$ | 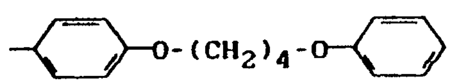 | 6-COOH | trans | 210° C |
| 10 | $-(CH_2)_2-CONHSO_2Ph$ | 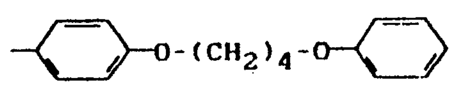 | 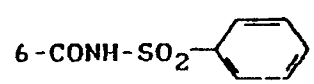 | trans | 0,61 (A) |

| Beispiel Nr. | A | B | G | Doppelbindungsisomer | Rf oder Schmelzpunkt (°C) |
|---|---|---|---|---|---|
| 11 | $-CH_3$ | 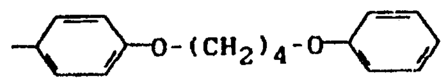 | 6-COOH | trans | 0,26 (C) |
| 12 | $-(CH_2)_2-COOH$ | 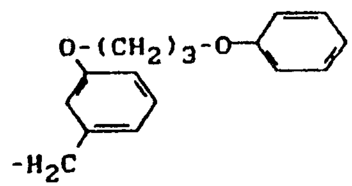 | H | cis | 0,34 (B) |
| 13 | $-(CH_2)_2-COOH$ | 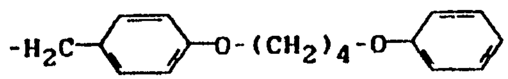 | 6-COOH | trans | 173° C |
| 14 | $-(CH_2)_2-COOH$ | 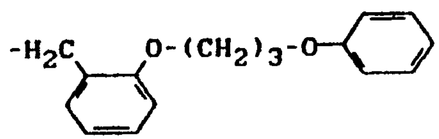 | 6-COOH | cis | 109° C |
| 15 | $-(CH_2)_2-COOH$ | 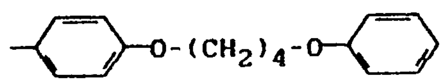 | 4-COOH | cis | 143° C |

FORTSETZUNG TABELLE 1

| Beispiel Nr. | A | B | G | Doppelbindungs-isomer | Rf, Schmelzpunkt (°C) oder Retentionszeit (Min.) |
|---|---|---|---|---|---|
| 16 | $-(CH_2)_2-COOH$ | 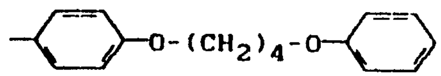 | 4-COOH | trans | 149-150° C |
| 17 | $-CH_2-COOH$ | 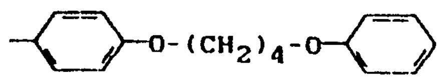 | 6-COOH | trans | 167° C |
| 18 | $-(CH_2)_2-COONa$ | 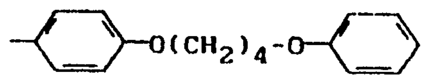 | 5-CN | trans | 8,44 (D) |
| 19 | $-(CH_2)_2-COONa$ | 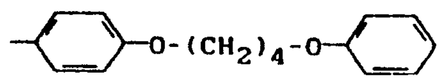 | 5-CN | cis | 8,43 (D) |
| 20 | $-(CH_2)_2-COONa$ | 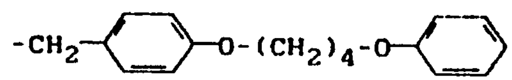 | 5-CN | trans | 8,56 (D) |
| 21 | $-(CH_2)_2-COONa$ | 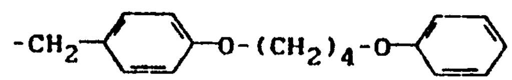 | 5-CN | cis | 8,63 (D) |
| 22 | $-(CH_2)_2-COONa$ | 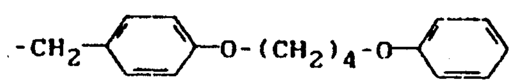 | 5 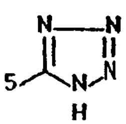 | cis | 0,52 (A) |

FORTSETZUNG TABELLE 1

| Beispiel Nr. | A | B | G | Doppelbindungsisomer | Rf, Schmelzpunkt (°C) oder Retentionszeit (Min.) |
|---|---|---|---|---|---|
| 23 | $-(CH_2)_2-COOH$ | 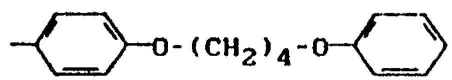 | 5-COOH | trans | 0,38 (A) |

Die folgenden Elutionsmittel wurden verwendet:

(A) = Dichlormethan/Methanol 9:1
(B) = Ethylacetat/Butanol/Essigsäure 9:1:0,1
(C) = Toluol/Ethylacetat 8:2
(D) = Nucleosil® 120-5 C 18, 5 µm, µ25 x 4 mm
10 bis 90 % Acetonitril, enthaltend 10 mM $H_3PO_4$, + 10 % Acetonitril,
Fliessgeschwindigkeit: 2 ml/Min., Raumtemperatur

**Ansprüche**

1. Indolderivate der allgemeinen Formel (I)

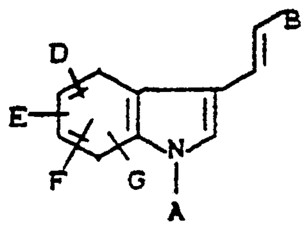

(I)

in der

A - ein geradkettiges oder verzweigtes Alkyl mit bis zu 12 Kohlenstoffatomen bezeichnet, das gegebenenfalls substituiert ist durch Cycloalkyl mit 3 bis 8 Kohlenstoffatomen, Halogen, Hydroxyl, Carboxyl, Cyano, geradkettiges oder verzweigtes Alkoxy oder Alkoxycarbonyl mit jeweils bis zu 10 Kohlenstoffatomen oder durch eine Gruppe der Formel $-CO-NH-SO_2-R^1$, in der

$R^1$ Aryl mit 6 bis 10 Kohlenstoffatomen bezeichnet, das gegebenenfalls einfach bis dreifach substituiert ist durch identische oder verschiedene Substituenten aus der Gruppe, umfassend Carboxyl, Halogen, Cyano, Nitro oder durch geradkettiges oder verzweigtes Alkyl, Alkoxy oder Alkoxycarbonyl mit jeweils bis zu 10 Kohlenstoffatomen,

oder durch Aryl mit 6 bis 10 Kohlenstoffatomen oder durch eine 5- bis 7-gliedrige gesättigte oder ungesättigte heterozyklische Gruppe mit bis zu 4 Heteroatomen, aus der Gruppe, umfassend Stickstoff, Schwefel oder Sauerstoff, die ihrerseits einfach bis vierfach substituiert sind durch identische oder verschiedene Substituenten aus der Gruppe, umfassend Carboxyl, geradkettiges oder verzweigtes Alkyl, Alkylthio, Alkoxy oder Alkoxycarbonyl mit jeweils bis zu 10 Kohlenstoffatomen, oder durch eine Gruppe der Formel $-NH-R^2$, in der

$R^2$ Wasserstoff, geradkettiges oder verzweigtes Alkyl oder Acyl mit jeweils bis zu 8 Kohlenstoffatomen bezeichnet,

B - geradkettiges oder verzweigtes Alkyl, Alkenyl oder Alkinyl mit 12 bis 18 Kohlenstoffatomen bezeichnet, das gegebenenfalls substituiert ist durch Halogen, Mercapto, geradkettiges oder verzweigtes Alkoxy, Acyl oder Alkoxycarbonyl mit jeweils bis zu 10 Kohlenstoffatomen oder durch Aryl mit 6 bis 10 Kohlenstoffatomen, das seinerseits einfach bis vierfach substituiert sein kann durch identische oder verschiedene Substituenten aus der Gruppe, umfassend Halogen, Nitro, Cyano, Carboxyl, Hydroxyl, oder durch eine Gruppe der Formel $-Y-(CH_2)_n-X-R^3$, in der

X und Y identisch oder verschieden sind, Sauerstoff oder Schwefel, oder eine direkte Bindung bezeichnen,

n eine Zahl 1, 2, 3, 4, 5 oder 6 bezeichnet und

$R^3$ Aryl mit 6 bis 10 Kohlenstoffatomen bezeichnet, das gegebenenfalls substituiert sein kann durch Hydroxyl, Carboxyl, Nitro, Cyano, Halogen oder durch geradkettiges oder verzweigtes Alkyl, Acyl, Alkoxy oder Alkoxycarbonyl mit jeweils bis zu 10 Kohlenstoffatomen,

oder

B - Aryl mit 6 bis 10 Kohlenstoffatomen bezeichnet, das gegebenenfalls einfach bis dreifach substituiert sein kann durch identische oder verschiedene Substituenten aus der Gruppe umfassend Halogen, Nitro, Hydroxyl oder durch eine Gruppe der Formel $-Y-(CH_2)_n-X-R^3$, in der

X, Y, $R^3$ und n die vorstehenden Bedeutungen haben,

D, E, F und G identisch oder verschieden sind und bezeichnen:

- Wasserstoff, Halogen, Cyano, Hydroxyl oder Carboxyl,
- geradkettiges oder verzweigtes Alkyl, Alkoxy, Alkoxycarbonyl oder Acyl mit jeweils bis zu 10 Kohlenstoffatomen oder Cycloalkyl mit 3 bis 8 Kohlenstoffatomen,
- eine 5- bis 7-gliedrige, gesättigte oder ungesättigte heterozyklische Gruppe mit bis zu 4 Heteroatomen aus der Gruppe, umfassend Stickstoff, Schwefel oder Sauerstoff, oder
- eine Gruppe der Formel $-CO-NH-SO_2-R^1$, in der

$R^1$ die vorerwähnte Bedeutung hat,

und deren physiologisch annehmbaren Salze.

2. Indolderivate gemäss Anspruch 1, in denen

A - geradkettiges oder verzweigtes Alkyl mit bis zu 10 Kohlenstoffatomen bezeichnet, das gegebenenfalls substituiert ist durch Cyclopropyl, Cyclopentyl, Cyclohexyl, Fluor, Chlor, Brom, Hydroxyl, Carboxyl, Cyano, geradkettiges oder verzweigtes Alkoxy oder Alkoxycarbonyl mit jeweils bis zu 8 Kohlenstoffatomen, oder durch eine Gruppe der Formel $-CO-NH-SO_2-R^1$, in der

$R^1$ Phenyl oder Naphthyl bezeichnet, wobei jede dieser Gruppen gegebenenfalls einfach oder zweifach substituiert ist durch identische oder verschiedene Substituenten aus der Gruppe, umfassend Carboxyl, Fluor, Chlor, Brom, Cyano, Nitro oder durch geradkettiges oder verzweigtes Alkyl, Alkoxy oder Alkoxycarbonyl mit jeweils bis zu 8 Kohlenstoffatomen,

oder durch Phenyl, Naphthyl, Thienyl, Furyl, Pyridyl, Pyrimidyl, Chinolyl, Isochinolyl, Pyrryl, Imidazolyl, 1,3-Thiazolyl, Tetrazolyl, Oxazolyl, Isoxazolyl, Isothiazolyl, Triazolyl oder Thiadiazolyl, die ihrerseits einfach bis dreifach substituiert sind durch identische oder verschiedene Substituenten aus der Gruppe, umfassend Carboxyl, geradkettiges oder verzweigtes Alkyl, Alkylthio, Alkoxy oder Alkoxycarbonyl mit jeweils bis zu 8 Kohlenstoffatomen, oder durch eine Gruppe der Formel $-NH-R^2$, in der

$R^2$ Wasserstoff oder geradkettiges oder verzweigtes Alkyl oder Acyl mit jeweils bis zu 6 Kohlenstoffatomen bezeichnet,

B - geradkettiges oder verzweigtes Alkyl oder Alkenyl mit jeweils 12 bis 16 Kohlenstoffatomen bezeichnet, wobei jedes gegebenenfalls substituiert ist durch Fluor, Chlor, Brom, Carboxyl, Hydroxyl, geradkettiges oder verzweigtes Alkoxy, Acyl oder Alkoxycarbonyl mit jeweils bis zu 8 Kohlenstoffatomen oder durch Phenyl, das seinerseits einfach bis dreifach substituiert sein kann durch identische oder verschiedene Substituenten aus der Gruppe, umfassend Fluor, Chlor, Brom, Carboxyl, Cyano, Hydroxyl, oder durch eine Gruppe der Formel $-Y-(CH_2)_n-X-R^3$, in der

X und Y identisch oder verschieden sind, Sauerstoff oder Schwefel, oder eine direkte Bindung bezeichnen,

n eine Zahl 1, 2, 3, 4, 5 oder 6 bezeichnet und

$R^3$ Phenyl oder Naphthyl bezeichnet, wobei jedes gegebenenfalls substituiert ist durch Hydroxyl, Carboxyl, Cyano, Fluor, Chlor, Brom, oder durch ein geradkettiges oder ein verzweigtes Alkoxy, Acyl oder Alkoxycarbonyl mit jeweils bis zu 8 Kohlenstoffatomen,

oder

B - Phenyl bezeichnet, das gegebenenfalls einfach oder doppelt substituiert ist durch identische oder verschiedene Substituenten aus der Gruppe umfassend Fluor, Chlor, Brom, Nitro, Hydroxyl oder durch eine Gruppe der Formel $-Y-(CH_2)_n-X-R^3$, in der

X, Y, n und $R^3$ die vorstehenden Bedeutungen haben,

D, E, F und G identisch oder verschieden sind und bezeichnen:

- Wasserstoff, Fluor, Chlor, Brom, Cyano, Carboxyl, geradkettiges oder verzweigtes Alkyl, Acyl oder Alkoxycarbonyl mit jeweils bis zu 8 Kohlenstoffatomen, Thienyl, Furyl, Pyridyl, Pyrimidyl, Chinolyl, Isochinolyl, Pyrryl, Imidazolyl, 1,3-Thiazolyl, Tetrazolyl, Oxazolyl, Isoxazolyl, Isothiazolyl, Triazolyl, Thiadiazolyl oder
- eine Gruppe der Formel $-CO-NH-SO_2-R^1$, in der

$R^1$ die vorerwähnte Bedeutung hat,

und deren physiologisch annehmbaren Salze.

3. Indolderivate gemäss Anspruch 1, in denen

A - geradkettiges oder verzweigtes Alkyl mit bis zu 8 Kohlenstoffatomen bezeichnet, das gegebenenfalls substituiert ist durch Carboxyl, Cyano, geradkettiges oder verzweigtes Alkoxycarbonyl mit bis zu 6 Kohlenstoffatomen oder durch eine Gruppe der Formel $-CO-NH-SO_2-R^1$, in der

$R^1$ Phenyl bezeichnet, das gegebenenfalls substituiert ist durch Cyano, Carboxyl oder durch geradkettiges oder verzweigtes Alkyl oder Alkoxycarbonyl mit jeweils bis zu 6 Kohlenstoffatomen,

oder durch Phenyl, Thienyl, Furyl, Pyrryl, 1,3-Thiadiazolyl, Tetrazolyl, Oxazolyl, Isoxazolyl, Isothiazolyl, Triazolyl oder Thiadiazolyl,

B - geradkettiges oder verzweigtes Alkyl oder Alkenyl mit 12 Kohlenstoffatomen bezeichnet, wobei jedes gegebenenfalls substituiert ist durch Carboxyl, Acetyl, Propanoyl, geradkettiges oder verzweigtes Alkoxy oder Alkoxycarbonyl mit jeweils bis zu 6 Kohlenstoffatomen, oder durch Phenyl, das seinerseits einfach oder zweifach substituiert sein kann durch identische oder verschiedene Substituenten aus der Gruppe, umfassend Cyano, Carboxyl oder durch eine Gruppe der Formel $-Y-(CH_2)_nX-R^3$, in der

X und Y identisch oder verschieden sind, Sauerstoff oder Schwefel oder eine direkte Bindung bezeichnen,

n eine Zahl 2, 3 oder 4 bezeichnet

und

$R^3$ Phenyl bezeichnet, das gegebenenfalls substituiert ist durch Hydroxyl, Cyano, Acetyl, Propanoyl, Carboxyl oder durch Alkoxycarbonyl mit bis zu 6 Kohlenstoffatomen, oder

B Phenyl bezeichnet, das substituiert ist durch eine Gruppe der Formel $-Y-(CH_2)_n-X-R^3$, in der

X, Y, n und $R^3$ die vorstehenden Bedeutungen haben,
D, E und F Wasserstoff bezeichnen, und
G - Cyano, Carboxyl, Alkoxycarbonyl mit bis zu 6 Kohlenstoffatomen, Thienyl, Furyl, Tetrazolyl, Triazolyl bezeichnet oder
- eine Gruppe der Formel $-CO-NH-SO_2-R^1$ bezeichnet, in der
$R^1$ die vorstehende Bedeutung hat,
und deren physiologisch annehmbaren Salze.

4. Indolderivate gemäss Anspruch 1, zur Behandlung von Erkrankungen.

5. Verfahren zur Herstellung von Indolderivaten der allgemeinen Formel (I)

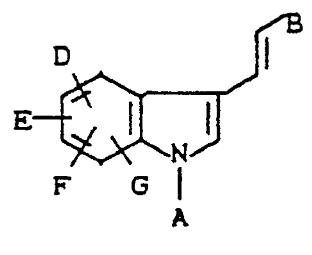

(I)

in der
A - ein geradkettiges oder verzweigtes Alkyl mit bis zu 12 Kohlenstoffatomen bezeichnet, das gegebenenfalls substituiert ist durch Cycloalkyl mit 3 bis 8 Kohlenstoffatomen, Halogen, Hydroxyl, Carboxyl, Cyano, geradkettiges oder verzweigtes Alkoxy oder Alkoxycarbonyl mit jeweils bis zu 10 Kohlenstoffatomen oder durch eine Gruppe der Formel $-CO-NH-SO_2-R^1$, in der
$R^1$ Aryl mit 6 bis 10 Kohlenstoffatomen bezeichnet, das gegebenenfalls einfach bis dreifach substituiert ist durch identische oder verschiedene Substituenten aus der Gruppe, umfassend Carboxyl, Halogen, Cyano, Nitro oder durch geradkettiges oder verzweigtes Alkyl, Alkoxy oder Alkoxycarbonyl mit jeweils bis zu 10 Kohlenstoffatomen,
oder durch Aryl mit 6 bis 10 Kohlenstoffatomen oder durch eine 5- bis 7-gliedrige gesättigte oder ungesättigte heterozyklische Gruppe mit bis zu 4 Heteroatomen, aus der Gruppe, umfassend Stickstoff, Schwefel oder Sauerstoff, die ihrerseits einfach bis vierfach substituiert sind durch identische oder verschiedene Substituenten aus der Gruppe, umfassend Carboxyl, geradkettiges oder verzweigtes Alkyl, Alkylthio, Alkoxy oder Alkoxycarbonyl mit jeweils bis zu 10 Kohlenstoffatomen, oder durch eine Gruppe der Formel $-NH-R^2$, in der
$R^2$ Wasserstoff, geradkettiges oder verzweigtes Alkyl oder Acyl mit jeweils bis zu 8 Kohlenstoffatomen bezeichnet,
B - geradkettiges oder verzweigtes Alkyl, Alkenyl oder Alkinyl mit 12 bis 18 Kohlenstoffatomen bezeichnet, das gegebenenfalls substituiert ist durch Halogen, Mercapto, geradkettiges oder verzweigtes Alkoxy, Acyl oder Alkoxycarbonyl mit jeweils bis zu 10 Kohlenstoffatomen oder durch Aryl mit 6 bis 10 Kohlenstoffatomen, das seinerseits einfach bis vierfach substituiert sein kann durch identische oder ver schiedene Substituenten aus der Gruppe, umfassend Halogen, Nitro, Cyano, Carboxyl, Hydroxyl, oder durch eine Gruppe der Formel $-Y-(CH_2)_n-X-R^3$, in der
X und Y identisch oder verschieden sind, Sauerstoff oder Schwefel, oder eine direkte Bindung bezeichnen,
n eine Zahl 1, 2, 3, 4, 5 oder 6 bezeichnet und
$R^3$ Aryl mit 6 bis 10 Kohlenstoffatomen bezeichnet, das gegebenenfalls substituiert ist durch Hydroxyl, Carboxyl, Nitro, Cyano, Halogen oder durch geradkettiges oder verzweigtes Alkyl, Acyl, Alkoxy oder Alkoxycarbonyl mit jeweils bis zu 10 Kohlenstoffatomen,
oder
B - Aryl mit 6 bis 10 Kohlenstoffatomen bezeichnet, das gegebenenfalls einfach bis dreifach substituiert ist durch identische oder verschiedene Substituenten aus der Gruppe umfassend Halogen, Nitro, Hydroxyl oder durch eine Gruppe der Formel $-Y-(CH_2)_n-X-R^3$, in der
X, Y, $R^3$ und n die vorstehenden Bedeutungen haben,
D, E, F und G identisch oder verschieden sind und bezeichnen:
- Wasserstoff, Halogen, Cyano, Hydroxyl oder Carboxyl,
- geradkettiges oder verzweigtes Alkyl, Alkoxy, Alkoxycarbonyl oder Acyl mit jeweils bis zu 10 Kohlenstoffatomen oder Cycloalkyl mit 3 bis 8 Kohlenstoffatomen,
- eine 5- bis 7-gliedrige, gesättigte oder ungesättigte heterozyklische Gruppe mit bis zu 4 Heteroatomen aus der Gruppe, umfassend Stickstoff, Schwefel oder Sauerstoff, oder

- eine Gruppe der Formel $-CO-NH-SO_2-R^1$, in der
$R^1$ die vorerwähnte Bedeutung hat,
und deren physiologisch annehmbaren Salze,
dadurch **gekennzeichnet,** dass man Aldehyde der allgemeinen Formel (II)

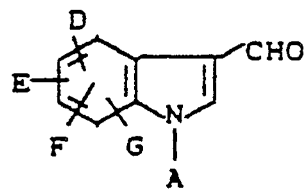

(II)

in der D, E, F, G und A die vorerwähnten Bedeutungen haben, mit Phosphorverbindungen der allgemeinen Formel (III), (IV) und (V)

$\overset{\oplus}{P}(R^4)_3CH_2B[Z]^{\ominus}$ (III)

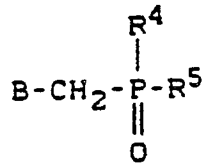

(IV)

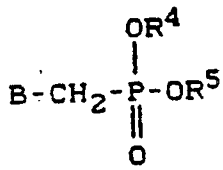

(V)

umsetzt in denen
B - die vorstehende Bedeutung hat,
$R^4$ und $R^5$ identisch oder verschieden sind und $(C_{1-6})$-Alkyl oder Phenyl bezeichnen, und
Z - Chlor, Brom, Jod oder das Tosylatanion bezeichnet,
in inerten Lösungsmitteln, geeignetenfalls in Anwesenheit von Basen und, im Falle der Säuren, durch Hydrolyse der Ester durch herkömmliche Methoden.

6. Medikamente, die wenigstens ein Indolderivat gemäss Anspruch 1 enthalten.
7. Verfahren zur Herstellung eines Medikamentes gemäss Anspruch 6, dadurch **gekennzeichnet,** dass Indolderivate gemäss Anspruch 1 in eine geeignete Form zur Darreichung gebracht werden, geeignetenfalls mit Hilfe herkömmlicher Hilfsstoffe und Träger.
8. Verwendung der Indolderivate gemäss Anspruch 1 zur Herstellung von Medikamenten.
9. Verwendung der Indolderivate gemäss Anspruch 1 zur Behandlung von Krankheiten.
10. Zwischenprodukte der allgemeinen Formel (II)

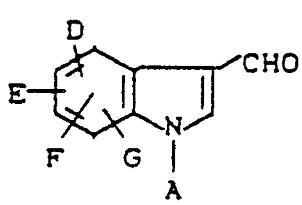

(II)

in der
A - ein geradkettiges oder verzweigtes Alkyl mit bis zu 12 Kohlenstoffatomen bezeichnet, das gegebenenfalls substituiert ist durch Cycloalkyl mit 3 bis 8 Kohlenstoffatomen, Halogen, Hydroxyl, Carboxyl, Cyano, geradkettiges oder verzweigtes Alkoxy oder Alkoxycarbonyl mit jeweils bis zu 10 Kohlenstoffatomen oder durch eine Gruppe der Formel $-CO-NH-SO_2-R^1$, in der
$R^1$ Aryl mit 6 bis 10 Kohlenstoffatomen bezeichnet, das gegebenenfalls einfach bis dreifach substituiert ist

durch identische oder verschiedene Substituenten aus der Gruppe, umfas send Carboxyl, Halogen, Cyano, Nitro oder durch geradkettiges oder verzweigtes Alkyl, Alkoxy oder Alkoxycarbonyl mit jeweils bis zu 10 Kohlenstoffatomen,

oder durch Aryl mit 6 bis 10 Kohlenstoffatomen oder durch eine 5- bis 7-gliedrige gesättigte oder ungesättigte heterozyklische Gruppe mit bis zu 4 Heteroatomen, aus der Gruppe, umfassend Stickstoff, Schwefel oder Sauerstoff, die ihrerseits einfach bis vierfach substituiert sind durch identische oder verschiedene Substituenten aus der Gruppe, umfassend Carboxyl, geradkettiges oder verzweigtes Alkyl, Alkylthio, Alkoxy oder Alkoxycarbonyl mit jeweils bis zu 10 Kohlenstoffatomen, oder durch eine Gruppe der Formel -NH-$R^2$, in der

$R^2$ Wasserstoff, geradkettiges oder verzweigtes Alkyl oder Acyl mit jeweils bis zu 8 Kohlenstoffatomen bezeichnet,

D, E, F und G identisch oder verschieden sind und bezeichnen:

- Wasserstoff, Halogen, Cyano, Hydroxyl oder Carboxyl,
- geradkettiges oder verzweigtes Alkyl, Alkoxy, Alkoxycarbonyl oder Acyl mit jeweils bis zu 10 Kohlenstoffatomen oder Cycloalkyl mit 3 bis 8 Kohlenstoffatomen,
- eine 5- bis 7-gliedrige, gesättigte oder ungesättigte heterozyklische Gruppe mit bis zu 4 Heteroatomen aus der Gruppe, umfassend Stickstoff, Schwefel oder Sauerstoff, oder
- eine Gruppe der Formel -CO-NH-$SO_2$-$R^1$, in der

$R^1$ die vorerwähnte Bedeutung hat.

11. Verfahren zur Herstellung von Zwischenprodukten gemäss der allgemeinen Formel (II)

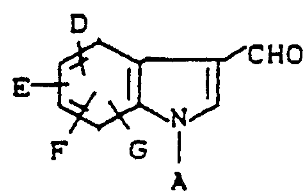

(II)

in der

A - ein geradkettiges oder verzweigtes Alkyl mit bis zu 10 Kohlenstoffatomen bezeichnet, das gegebenenfalls substituiert ist durch Cyclopropyl, Cyclopentyl, Cyclohexyl, Fluor, Chlor, Brom, Hydroxyl, Carboxyl, Cyano, geradkettiges oder verzweigtes Alkoxy oder Alkoxycarbonyl mit jeweils bis zu 8 Kohlenstoffatomen oder durch eine Gruppe der Formel -CO-NH-$SO_2$-$R^1$, in der

$R^1$ Aryl mit 6 bis 10 Kohlenstoffatomen bezeichnet, das gegebenenfalls einfach bis dreifach substituiert ist durch identische oder verschiedene Substituenten aus der Gruppe, umfassend Carboxyl, Halogen, Cyano, Nitro oder durch geradkettiges oder verzweigtes Alkyl, Alkoxy oder Alkoxycarbonyl mit jeweils bis zu 10 Kohlenstoffatomen,

oder durch Aryl mit 6 bis 10 Kohlenstoffatomen oder durch eine 5- bis 7-gliedrige gesättigte oder ungesättigte heterozyklische Gruppe mit bis zu 4 Heteroatomen, aus der Gruppe, umfassend Stickstoff, Schwefel oder Sauerstoff, die ihrerseits einfach bis vierfach substituiert sind durch identische oder verschiedene Substituenten aus der Gruppe, umfassend Carboxyl, geradkettiges oder verzweigtes Alkyl, Alkylthio, Alkoxy oder Alkoxycarbonyl mit jeweils bis zu 10 Kohlenstoffatomen, oder durch eine Gruppe der Formel -NH-$R^2$, in der

$R^2$ Wasserstoff, geradkettiges oder verzweigtes Alkyl oder Acyl mit jeweils bis zu 8 Kohlenstoffatomen bezeichnet,

D, E, F und G identisch oder verschieden sind und bezeichnen:

- Wasserstoff, Halogen, Cyano, Hydroxyl oder Carboxyl,
- geradkettiges oder verzweigtes Alkyl, Alkoxy, Alkoxycarbonyl oder Acyl mit jeweils bis zu 10 Kohlenstoffatomen oder Cycloalkyl mit 3 bis 8 Kohlenstoffatomen,
- eine 5- bis 7-gliedrige, gesättigte oder ungesättigte heterozyklische Gruppe mit bis zu 4 Heteroatomen aus der Gruppe, umfassend Stickstoff, Schwefel oder Sauerstoff, oder
- eine Gruppe der Formel -CO-NH-$SO_2$-$R^1$, in der

$R^1$ die vorerwähnte Bedeutung hat,

dadurch **gekennzeichnet,** dass man Verbindungen der allgemeinen Formel (VI)

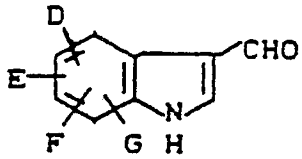

(VI)

in der in der D, E, F und G die vorstehenden Bedeutungen haben, mit Verbindungen der allgemeinen Formel (VII)

Hal-A (VII)

umsetzt, in der Hal Fluor, Chlor, Brom oder Jod bezeichnet und A die vorerwähnte Bedeutung hat, in inerten Lösungsmitteln, geeignetenfalls in Anwesenheit einer Base und, im Falle der Säuren, die Ester durch die vorerwähnten Methoden hydrolysiert.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 90113024.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 251 011 (BAYER AG KONZERNVERWALTUNG RP PATENTABTEILUNG) * Patentansprüche 1,7 * -- | 1,6 | C 07 D 209/22<br>C 07 D 209/24<br>A 61 K 31/40 |
| D,A | US - A - 4 783 479 (DOMINICK MOBILIO) * Spalten 1,2 * ---- | 1 | |

RECHERCHIERTE SACHGEBIETE (Int. Cl.4)

C 07 D 209/00
A 61 K

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:
Unvollständig recherchierte Patentansprüche:
Nicht recherchierte Patentansprüche: 9
Grund für die Beschränkung der Recherche: Verfahren zur therapeutischen Behandlung des menschlichen oder tierischen Körpers, Artikel 52 (4)

| Recherchenort | Abschlußdatum der Recherche | Prüfer |
|---|---|---|
| VIENNA | 03-09-1990 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03.82